# EUROPÄISCHE PATENTANMELDUNG

(11) **EP 2 650 024 A1**
(43) Veröffentlichungstag der Anmeldung: **16.10.2013**
(21) Anmeldenummer: 13001627.2
(22) Anmeldetag: 28.03.2013
(51) Int. Cl.: A61L 9/14, A61L 9/03, A01M 1/20

(54) **Verfahren und Vorrichtung zur molekularen Verdunstung von Duft-/Wirkstoffen und deren Ausbreitung**

(30) Priorität: 12.04.2012 DE 102012007167
(71) Anmelder: ORTNER, Georg, 50668 Köln (DE); Linde AG, 80331 München (DE)
(72) Erfinder: Ortner, Georg, 50668 Köln (DE)
(74) Vertreter: Grättinger Möhring von Poschinger Patentanwälte Partnerschaft

(57) **Zusammenfassung**

Bei einem Verfahren zur molekularen Verdunstung von Duft- und/oder Wirkstoffen (DW-Stoffen) und deren Ausbreitung über Luftströme in der umgebenden Luft, in geschlossenen Räumen, in Kanälen von Lüftungs- und Klimaanlagen oder dergleichen, wobei DW-Stoff vor dessen Ausbreitung gezielt auf einen bandförmigen DW-Stoff-Träger (6) flüssig aufgebracht wird, ist vorgesehen,
dass der bandförmige DW-Stoff-Träger (6) umlaufend, wenigstens teilweise innerhalb einer Ausbreitungszone für DW-Stoff, bewegt wird und
dass der zu verdunstende DW-Stoff während seines Umlaufs auf dem DW-Stoff-Träger (6) direkt oder indirekt auf eine die molekulare Verdunstung fördernde Temperatur aufgewärmt wird.

## Beschreibung

Die Erfindung betrifft ein Verfahren und eine Vorrichtung gemäß den Oberbegriffen der unabhängigen Patentansprüche.

In einer Internationalen Patentanmeldung mit Veröffentlichungsnummer WO 2011/054529 A1 sind ein Verfahren und eine Vorrichtung zur Erzeugung eines Duftluftstroms zur Beduftung von Räumen, Objekten oder dergleichen beschrieben, wobei der Duftstoff auf einen bandförmigen Duftstoffträger gezielt aufgespritzt wird, der zwecks Verdunstung des Duftstoffs durch einen Verdunstungsschacht hindurch bewegt wird, welcher von Frischluft durchströmt ist. Nach seinem Austreten aus dem Verdunstungsschacht wird der benutzte Abschnitt des Duftstoffträgers als Abfall beseitigt. Der besondere Vorteil hierbei ist, dass die Zumischung von Duftstoff in den Frischluftstrom ohne unerwünschte Ablagerungen flüssiger Duftstoffe auf Teilen der Vorrichtung erfolgen kann, sodass jede Verschmutzung bzw. Versottung der Vorrichtung unterbleibt. Vielmehr wird der Duftstoff durch die im Verdunstungsschacht strömende Frischluft in molekularer Form nach vorherigem Verdunsten des in flüssiger Form auf den Duftstoffträger aufgebrachten Duftstoffs aufgenommen. Der Verdunstungsvorgang kann dabei durch Zuführung von erwärmter Frischluft noch verbessert werden, d.h. es wird dadurch eine weitgehende Verdunstung in kürzerer Zeit erzielt.

Die Beduftung von Räumen kann dabei weitgehend automatisch ablaufen, wobei mittels einer elektronischen Steuereinrichtung die pro Zeiteinheit verdunstete Duftstoffmenge nach Programmvorgaben veränderbar ist.

Demgegenüber liegt der vorliegenden Erfindung die Aufgabe zugrunde, ein Verfahren und eine Vorrichtung zu schaffen, bei welchen der Verdunstungsvorgang besonders effektiv und kostensparend abläuft, wobei er automatisch nach Programmvorgaben steuerbar ist und wobei die Ausbreitung von Duft-und/oder Wirkstoffen auf besonders effektive Weise erfolgt, gleichgültig ob im freien Luftzug, z.B. unter Konvektion oder über Klima- und Lüftungsanlagen, und auch noch bei relativ hohen Strömungsgeschwindigkeiten der anströmenden Frischluft.

Diese Aufgabe wird erfindungsgemäß nach einem Verfahren gemäß den Kennzeichen der unabhängigen Ansprüche gelöst.

Hierzu ist bei einem Verfahren zur molekularen Verdunstung von Duft- und/oder Wirkstoffen (DW-Stoffen) und deren Ausbreitung über Luftströme in der umgebenden Luft, in geschlossenen Räumen, in Kanälen von Lüftungs- und Klimaanlagen oder dergleichen, wobei DW-Stoff vor dessen Ausbreitung gezielt auf einen bandförmigen DW-Stoff-Träger flüssig aufgebracht wird und wobei der mit DW-Stoff beladene DW-Stoff-Träger quer zu seiner Längsachse von einem Luftstrom angeströmt wird, vorgesehen,
dass der bandförmige DW-Stoff-Träger umlaufend, wenigstens teilweise innerhalb einer Ausbreitungszone für DW-Stoff, bewegt wird und
dass der zu verdunstende DW-Stoff während seines Umlaufs auf dem DW-Stoff-Träger direkt oder indirekt auf eine die molekulare Verdunstung fördernde Temperatur aufgewärmt wird

Mit diesem Verfahren gelingt es, einen mit DW-Stoff beladenen Luftstrom beliebigen Volumens kontinuierlich und in immer gleichbleibender Qualität bei genau dosierbarer Quantität des molekular verdunsteten DW-Stoffs, d.h. mit vorgegebener DW-Stoff-Intensität, zu erzeugen. Bei kontrollierter, gleichbleibender Verdunstung ergibt sich ein z.B. nach einem vorgegebenen Programm mit DW-Stoff angereicherter Luftstrom mit gleichbleibender Qualität, unabhängig davon, ob die Antrömung des umlaufenden DW-Stoff-Trägers im Saugstrom oder im Blasstrom der zu behandelnden Frischluft erfolgt. Je nach Strömungsvolumen der Frischluft kann die Verdunstungsoberfläche angepasst werden von wenigen mm² für kleinere Räume bis zu mehreren m², z.B. für die kontinuierliche Beduftung von Landschaften oder deren Behandlung mit Wirkstoffen.

Ein sparsamer Verbrauch von DW-Stoffen wird dadurch gewährleistet, dass der DW-Stoff-Träger direkt oder indirekt auf eine die Verdunstung fördernde Temperatur aufgewärmt wird. Die Wärme kann dabei mehr oder weniger direkt auf den DW-Stoff-Träger übertragen werden. Auch eine indirekte Aufheizung mittels eines Teilluftstroms in unmittelbarer Umgebung des DW-Stoff-Trägers ist denkbar.

Besonders vorteilhaft ist eine auf Rollen umlaufende Bewegung des bandförmigen DW-Stoff-Trägers innerhalb der Ausbreitungszone. Wichtige Anwendungsfälle sind dabei die DW-Stoff-Einbringung in Türluftschleiern z.B. von Hoteleingängen, Eingängen von Geschäften, Messehallen oder dergleichen. Hierbei kommt es besonders auf eine gleichmäßige Duftverteilung über die gesamte Türbreite an, die vorteilhaft mittels eines bandförmigen DW-Stoff-Trägers, welcher zwischen Umlenkrollen umläuft, auf zuverlässige Weise verwirklicht werden kann. Auch die Raumbeduftung mittels DW-Stoffen spielt eine zunehmend größere Rolle, z.B. der Geschäftsräume von Markenartiklern, Modegeschäften oder sonstigen Räumen für Werbeveranstaltungen, Ausstellungen usw..

Bei diesen Anwendungsfällen erstreckt sich die Ausbreitungszone beispielsweise über die gesamte Breite eines Raumzugangs, einer Eingangstür oder dergleichen. Erfindungsgemäß ist dabei anzustreben, dass zum Erzielen einer gleichmäßigen DW-Stoff-Beaufschlagung des zu erfassenden Luftstroms die Umlauflänge des auf Rollen bewegten DW-Stoff-Trägers möglichst weitgehend der Breite des Strömungsdurchgangs angepasst wird.

Zur Steuerung der DW-Stoff-Intensität ist erfindungsgemäß vorgesehen, dass der DW-Stoff-Träger mit variabel einstellbarer Geschwindigkeit bewegbar ist bzw. dass die Menge des auf den DW-Stoff-Träger in flüssiger Form aufgebrachten DW-Stoffs einstellbar ist. In diesem Sinne besteht erfindungsgemäß die Möglichkeit, den DW-Stoff-Träger durch entsprechende Programmierung einer Dosiervorrichtung stets mit frischem DW-Stoff zu versorgen, wobei zeitliche Intervalle und Dosierstoffmengen durch variabel einstellbar sind.

Zur Regelung der Verdunstungstemperatur ist in Ausgestaltung des erfindungsgemäßen Verfahrens vorgesehen, dass durch direkte oder indirekte Aufheizung des DW-Stoff-Trägers eine geeignete Temperatur im Bereich zwischen 40° und 100° C wählbar ist. Bevorzugt kommt eine Verdunstungstemperatur von ca. 60° - 80° C zur Anwendung. Im Falle von DW-Stoffen, welche kristalline Substanzen enthalten, kommen noch wesentlich höhere Temperaturen in Frage.

Eine räumlich möglichst über die gesamte Breite des zu behandelnden Luftstroms erstreckte Heizwirkung ist im Interesse einer vollständigen Verdunstung des aufgebrachten DW-Stoffs vorteilhaft, d.h. nicht nur die räumliche Ausdehnung des umlaufenden DW-Stoff-Trägers sondern auch der diesem zugeordneten Heizvorrichtung ist für die Effektivität des erfindungsgemäßen Verfahrens von Bedeutung.

Eine erfindungsgemäße Vorrichtung zur Durchführung des vorbeschriebenen Verfahrens zur molekularen Verdunstung und Ausbreitung von Duft-/Wirkstoffen über Luftströme in der umgebenden Luft, in geschlossenen Räumen, in Kanälen von Lüftungs- und Klimaanlagen oder dergleichen umfasst eine Dosiervorrichtung zum Aufbringen von DW-Stoffen in flüssiger Form auf einen bandförmigen DW-Stoff-Träger, der wenigstens teilweise innerhalb einer Ausbreitungszone bewegt und dabei quer zu seiner Längsachse von einem Luftstrom angeströmt wird, und wobei die Dosiervorrichtung außerhalb der Ausbreitungszone angeordnet ist.

Die erfindungsgemäße Vorrichtung umfasst insbesondere einen bandförmigen DW-Stoff-Träger, der an einem Tragprofil auf Rollen umlaufend bewegbar ist sowie eine Heizvorrichtung, welche sich im Wesentlichen längs der Bewegungsbahn des DW-Stoff-Trägers erstreckt.

Im Ergebnis ist damit eine vollständige Verdunstung des aufgebrachten DW-Stoffs und dessen gleichmäßige Verteilung über die gesamte Breite einer Ausbreitungszone erzielbar, ohne dass dabei der zu behandelnde Luftstrom insgesamt aufgeheizt wird. Vielmehr genügt es, den DW-Stoff-Träger aufzuheizen, d.h. auf eine die Verdunstung begünstigende Temperatur zu bringen.

Dies kann gemäß einer weiteren Ausgestaltung besonders energiesparend dadurch erfolgen, dass der bandförmige, auf Rollen umlaufende DW-Stoff-Träger sowie die zugeordnete Heizvorrichtung wenigstens innerhalb der Ausbreitungszone bis auf Be- und Entlüftungsöffnungen gekapselt angeordnet sind.

Hierzu eignet sich besonders vorteilhaft eine Heizvorrichtung in Form eines lang gestreckten Heizstabs, welcher innerhalb der Kapsel zwischen einem oberen und einem unteren Trum des zwischen zwei Umlenkrollen umlaufenden DW-Stoff-Trägers angeordnet ist.

Der Heizstab strahlt dabei seine Wärme auf den umlaufenden DW-Stoff-Träger über dessen gesamte Länge gleichmäßig ab, sodass es zur unmittelbaren Verdunstung des auf dem DW-Stoff-Träger in flüssiger Form aufgebrachten DW-Stoffs kommt. Ein durch die Belüftungsöffnungen eindringender geringer Frischluftanteil wird in der Kapsel verwirbelt und sorgt für eine Verdunstung des DW-Stoffs in molekularer Form und dessen Ausbreitung durch Entlüftungsöffnungen aus der Kapsel heraus in die außen vorbei strömende Luftströmung.

Als Kapsel eignet sich besonders vorteilhaft ein Hohlprofil, welches an gegenüber liegenden Seiten schmale Schlitzöffnungen aufweist, einerseits für den Eintritt von Frischluft in die Kapsel, andererseits für deren Austritt mit den aufgenommenen DW-Stoffmolekülen, sodass diese sich in dem umgebenden Luftstrom verteilen.

Das Hohlprofil ist vorteilhaft aus Teilprofilen zusammengesetzt, welche zwischen sich schmale Schlitze begrenzen bzw. ein oder mehrere schlitzförmige Öffnungen aufweisen.

Vorteilhaft sind die Teilprofile in einem U-förmigen Außenprofil aufgenommen, welches ein- oder beidseitig strömungsgünstige Profilansätze aufweist. Diese dienen der Erzeugung einer Sogwirkung auf einer Außenseite des Hohlprofils welcher die Schlitzöffnungen zugeordnet sind.

Um Gewicht zu sparen bestehen die Teilprofile und das Außenprofil zweckmäßig aus Leichtmetall, z.B. aus Aluminium.

Zwischen den Teilprofilen und dem diese einfassenden Außenprofil ist ein U-Profil aus wärmeisolierendem Material eingesetzt. Dieses dient der Speicherung der vom Heizstab im Inneren des Hohlprofils erzeugten Wärme bei gleichzeitiger Minimierung von Energieverlust. Alternativ kann auch das Außenprofil selbst aus Wärme isolierendem Material bestehen. Das genannte U-Profil kann sich dabei erübrigen.

Ein geeigneter DW-Stoff-Träger besteht aus einem aus Einzelfäden eng gebundenen Metallband mit entsprechend hoher Kapillarwirkung. Ein derartiges Metallband, welches beispielsweise in Art eines Flechtbands aus geflochtenen Metallfäden gebildet ist, besitzt eine hohe Lebensdauer; durch die ihm eigene Kapillarwirkung nimmt es die flüssig aufgebrachten DW-Stoffe tropfenfrei auf und ermöglicht deren problemlose Verdunstung, gefördert durch die Wärmeeinwirkung des in unmittelbarer Nähe des DW-Stoff-Trägers angeordneten Heizstabs.

Vor einem Wechsel des DW-Stoffs kann ein derartiges Metallband entweder gereinigt oder nach längerem Gebrauch ausgetauscht werden.

Für einen zwischen zwei Umlenkrollen umlaufenden DW-Stoff-Träger, der je nach Ausdehnung der Ausbreitungszone eine Länge von 0,2 bis zu mehreren Metern haben kann, ist vorteilhaft, dass eine der Umlenkrollen außerhalb der Ausbreitungszone als Antriebsrolle ausgebildet ist, die von einem daran angekoppelten Elektromotor angetrieben wird.

Wie vorstehend beschrieben sind zwei wesentliche Bauteile des Erfindungsgegenstands nämlich der umlaufende DW-Stoff-Träger und die zugehörige Heizvorrichtung innerhalb eines der Kapselung dieser Bauteile dienenden Hohlprofils aufgenommen. Bei der Montage wird die erfindungsgemäße Vorrichtung vorteilhaft derart an einem Lüftungskanal befestigt, dass dessen Hohlprofil durch eine Wandöffnung des Lüftungskanals in dessen Inneres in Art einer Lanze frei hinein ragt und nur mit einem Ende an der Wand des Lüftungskanals befestigt wird. Auf der Außenseite der Wand befinden sich weitere Bauteile der erfindungsgemäßen Vorrichtung, wie z.B. eine äußere angetriebene Umlenkrolle mit Antriebsmotor, die Dosiervorrichtung, die elektronische Steuerung sowie ein DW-Stoff-Vorratsbehälter.

Die Einbaulage des Hohlprofils kann beliebig zwischen einer waagrechten und einer vertikalen Position bevorzugt in einer Ebene quer zur Längsrichtung des im Lüftungskanal geführten Luftstroms üblicherweise also quer zum Lüftungskanal, gewählt werden.

Neben Lüftungskanälen von Lüftungs- und Klimaanlagen kommen als bevorzugtes Anwendungsgebiet auch Zuluftkanäle für Türluftschleier in Frage. Hierbei kann die erfindungsgemäße Vorrichtung auch unmittelbar vor dem Ansaugbereich der den Türluftschleier erzeugenden Einheit, z.B. in einem gesonderten Gehäuse aus Lochblech angeordnet werden.

In diesem Sinne ist in weiterer Ausgestaltung der vorliegenden Erfindung vorgesehen, dass bei einer Vorrichtung zum Erzeugen eines Türluftschleiers die erfindungsgemäße Vorrichtung ganz oder teilweise außerhalb oder innerhalb des Strömungskanals für die Zuluft angeordnet ist und von dieser quer zu Ihrer Längsachse geströmt wird.

Im Folgenden wird ein Ausführungsbeispiel der erfindungsgemäßen Vorrichtung anhand der Zeichnung beschrieben. Es zeigt
- Fig. 1: eine schematische Systemansicht der Verdunstungsvorrichtung,
- Fig. 2: einen Längsschnitt durch die Verdunstungsvorrichtung in gekapselter Form und
- Fig. 3: einen Querschnitt durch die Verdunstungsvorrichtung gemäß III-III der Figur 2.

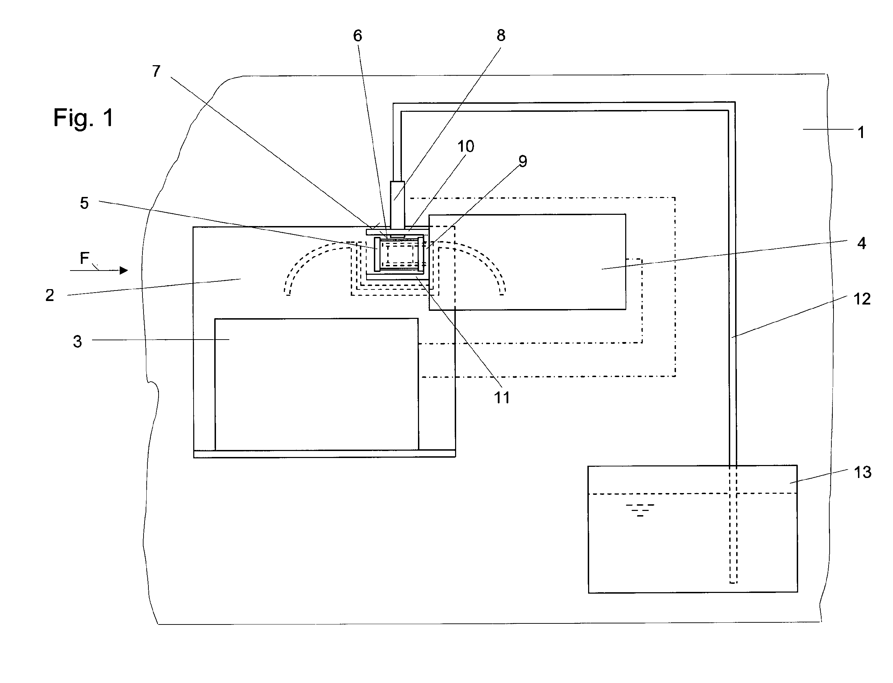

Fig. 1 zeigt eine schematische Ansicht der Verdunstungsvorrichtung mit Blick auf die Außenseite einer Seitenwand 1 eines Lüftungskanals, auf welcher eine Montageplatte 2 mit den darauf aufgebauten Teilen der Verdunstungsvorrichtung befestigt ist. Dazu zählen eine elektronische Steuereinheit 3, ein Elektromotor 4, eine DW-Stoff-Düse 8 einer Dosiervorrichtung, welche über eine DW-Stoff-Versorgungsleitung 12, die als Druck- oder Saugleistung wirken kann, mit einem Vorratsbehälter 13 verbunden ist, in welchem sich DW-Stoff in flüssiger Form befindet.

Der Motor 4 dient zum Antrieb einer außerhalb des Lüftungskanals angeordneten angetriebenen Umlenkrolle 5 für den Umlauf eines bandförmigen DW-Stoff-Trägers 6, auf welchen DW-Stoff in flüssiger Form mittels der Düse 8 aufgebracht wird. Sowohl die Düse 8 als auch der Elektromotor 4 sind an einem außerhalb der Seitenwand 1 befindlichen Ende eines U-Profils 7 befestigt, in dessen Innerem der umlaufende DW-Stoffträger 6 aufgenommen ist. Der Elektromotor 4 und die Düse 8 sind mit dem U-Profil 7 fest verbunden. Die Antriebswelle zwischen dem Elektromotor 4 und der angetriebenen Umlenkrolle 5 ist nicht eingezeichnet, sie erstreckt sich vom Elektromotor 4 durch den Quersteg 9 des U-Profils 7 hindurch, wo sie mit der Umlenkrolle 5 drehfest verbunden ist. Die beiden Schenkel 10, 11 des U-Profils 7 weisen entgegen die Anströmrichtung gemäß Pfeil F der Luftströmung im Inneren des Lüftungskanals, sodass ein geringer Strömungsanteil auf der offenen Seite des U-Profils 7 in dessen Inneres gelangen und dort den DW-Stoff-Träger 6 von allen Seiten umspülen kann.

Die Düse 8 sitzt im oberen Schenkel 10 des U-Profils 7, welches bevorzugt als AL-Profil ausgebildet ist.

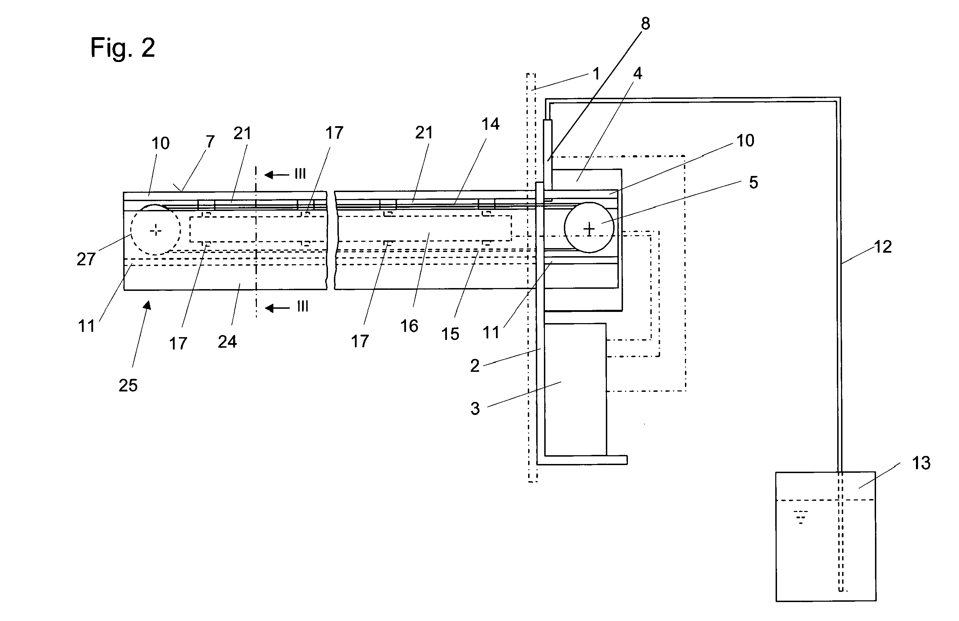

Die Steuereinheit 3 ist über mit strichpunktierten Linien angedeutete Steuerleitungen mit dem Elektromotor 4, der Düse 8 und mit einem Heizstab 16 (Fig. 2) verbunden, sodass deren automatische Betätigung über ein Computerprogramm gesteuert mittels entsprechender Sensoren, welche u. a. die Dosierung überwachen, möglich ist.

In Fig. 2 ist auch der innerhalb des Lüftungskanals angeordnete Teil der Verdunstungsvorrichtung gezeichnet. Die außerhalb der Seitenwand 1 des Lüftungskanals befindlichen Vorrichtungsteile sind mit denselben Bezugszeichen wie in Fig. 1 versehen.

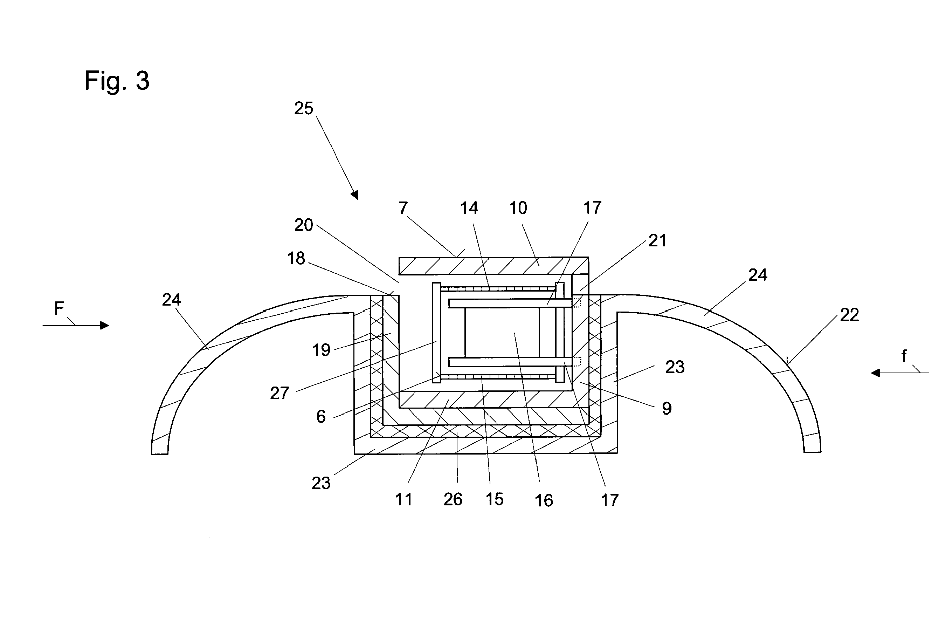

Das U-Profil 7 ist Teil einer Kapselung des stabförmig in das Innere des Lüftungskanals vorspringenden Hohlprofilträgers 25 der Verdunstungsvorrichtung. Dieser Hohlprofilträger 25 ist in vergrößerter Darstellung in Fig. 3 gezeichnet, welche einem Schnitt in der Ebene III-III der Figur 2 entspricht. In Fig. 2 ist mit strichlierten Linien das in Fig. 3 deutlich vergrößerte Schnittbild des Hohlprofilträgers 25 dargestellt. Im Inneren des U-Profils 7 befindet sich neben dem bandförmigen DW-Stoff-Träger 6 mit oberem Trum 14 und unterem Trum 15 ein Heizstab 16, der zwischen oberen und unteren Haltebolzen 17 am Quersteg 9 des U-Profils 7 befestigt ist.

Das U-Profil 7 ist auf einem L-Profil 18 (Fig. 3) aufgelagert, derart, dass dessen zum Quersteg 9 paralleler Schenkel 19 das U-Profil 7 auf der offenen Seite weitgehend schließt. Zwischen dem Ende des Schenkels 19 des L-Profils 18 und dem benachbarten Ende des oberen Schenkels 10 des U-Profils 7 verbleibt nur ein schmaler Spalt 20 (Fig. 3), durch welchen ein geringer Anteil der Luftströmung aus der Richtung gemäß Pfeil F im Inneren des Lüftungskanals in das Innere des U-Profils 7 eindringt, dort den bandförmigen DW-Stoff-Träger 6 von allen Seiten umspült und aus dem U-Profil 7 durch schmale Schlitze 21 in dessen Quersteg 9 wieder austritt. Auf diese Weise gelangen die im Inneren des U-Profils 7 durch Verdunsten gebildeten DW-Stoff-Moleküle in die Luftströmung im Inneren des Lüftungskanals. Dort werden die DW-Stoff-Moleküle bei Vermeidung von Tröpfchenkontamination von der Luftströmung aufgenommen und in die Ausbreitungszone transportiert.

Zur Förderung der Strömung im Inneren des U-Profils 7 ist noch ein Außenprofil 22 vorgesehen, welches zusammengesetzt ist aus einem mittleren U-Profilteil 23 und seitlichen Profilansätzen 24, welche eine seitliche Wölbung beschreiben, um auf diese Weise die anströmende Luft entsprechend der Wölbung in Richtung auf die Oberseite des stabförmigen Hohlprofilträgers 25 umzulenken, sodass die Spülung des Innenraums des U-Profils 7 durch Erzeugung eines Unterdrucks gefördert wird. Die gewölbten Profilansätze 24 des Außenprofils 22 finden sich auf beiden Seiten des mittleren U-Profilteils 23, damit etwa gleiche Strömungsverhältnisse bei einer Umkehr der Durchströmungsrichtung (vgl. Pfeil f) vorherrschen. Zur Verbesserung der Wärmeisolierung im Inneren des U-Profils 7 besitzt der in Fig. 3 dargestellte Profilquerschnitt auch noch ein der Wärmeisolierung dienendes U-Profil 26 aus einem wärmebeständigen Kunststoff. Auf diese Weise und durch die enge Kapselung des umlaufenden DW-Stoff-Trägers 6 und des zwischen seinem oberen Trum 14 und seinem unteren Trum 15 angeordneten Heizstabs 16 im Inneren des U-Profils 7 gelingt es, den Energieaufwand für den Betrieb des Heizstabs 16 zu minimieren.

Die Montage der Verdunstungsvorrichtung wird dadurch erheblich vereinfacht, dass anstelle eines geschlossenen Hohlprofils ein Hohlprofilträger 25 aus mehrteilig zusammengesetzten Profilen 7, 18, 26, 23 gewählt wird. Dadurch können in das offene U-Profil 7 zuerst die Einbauten in dessen Innerem, vorgenommen werden, wonach das U-Profil 7 mit den Einbauten mit den restlichen Profilteilen 18, 26, 23 auf einfache Weise verbunden werden kann.

Über die in Fig. 3 dargestellten Bauteile hinaus ist es noch erforderlich, im inneren Endbereich des stabförmigen Hohlprofilträgers 25 die zweite Umlenkrolle 27 zu montieren, was ebenfalls bei offenem U-Profil 7 im Wesentlichen behinderungsfrei erfolgen kann.

Mit Ausnahme des wärmeisolierenden U-Profils 26 können alle übrigen Profile zweckmäßig als Aluminiumprofile ausgebildet sein. Als Bandmaterial für den umlaufenden DW-Stoff-Träger 6 kommt bevorzugt ein Geflecht aus dünnen Metallfäden in Frage, welches den Vorteil bietet, dass es eine hohe Kapazität für die Aufnahme von flüssigem DW-Stoff besitzt, sodass sich beim Auftragen keinerlei Flüssigkeitsspiegel aufbaut; stattdessen wird die auf das Metallband dosiert aufgebrachte Flüssigkeit verzögerungsfrei in die Geflechthohlräume des Metallbandes durch Kapillarwirkung eingesogen. Das Metallband hat überdies den Vorteil, dass es ohne großen Aufwand nach längerem Gebrauch gereinigt werden kann.

Durch die besonders vorteilhafte Kapillarwirkung des aus Metallfäden gebundenen Metallbands kommen auch vertikale Einbaulagen ohne Gefahr eines Abtropfens in Frage. Der Wartungsaufwand für die im Inneren des Lüftungskanals positionierten Vorrichtungsteile ist somit gering. Gleiches gilt für die an der Außenseite des Lüftungskanals montierten Vorrichtungsteile, welche ohnehin nachteiligen Einwirkungen durch die Luftströmung entzogen sind und damit eine gute Zugänglichkeit für Wartungszwecke gewährleisten.

## Patentansprüche

1. Verfahren zur molekularen Verdunstung von Duft- und/oder Wirkstoffen (DW-Stoffen) und deren Ausbreitung über Luftströme in der umgebenden Luft, in geschlossenen Räumen, in Kanälen von Lüftungs- und Klimaanlagen oder dergleichen, wobei DW-Stoff vor dessen Ausbreitung gezielt auf einen bandförmigen DW-Stoff-Träger (6) flüssig aufgebracht wird, wobei der mit DW-Stoff beladene DW-Stoff-Träger quer zu seiner Längsachse von einem Luftstrom angeströmt wird,
**dadurch gekennzeichnet,**
**dass** der bandförmige DW-Stoff-Träger (6) umlaufend, wenigstens teilweise innerhalb einer Ausbreitungszone für DW-Stoff, bewegt wird und dass der zu verdunstende DW-Stoff während seines Umlaufs auf dem DW-Stoff-Träger (6) direkt oder indirekt auf eine die molekulare Verdunstung fördernde Temperatur aufgewärmt wird.

2. Verfahren nach Anspruch 1,
**dadurch gekennzeichnet,**
**dass** der DW-Stoff-Träger (6) auf Rollen umlaufend bewegt wird.

3. Verfahren nach Anspruch 1,
**dadurch gekennzeichnet,**
**dass** der DW-Stoff-Träger (6) mit variabel einstellbarer Geschwindigkeit bewegt wird.

4. Verfahren nach Anspruch 1,
**dadurch gekennzeichnet,**
**dass** die Menge des auf den DW-Stoff-Träger (6) aufgebrachten DW-Stoffs einstellbar ist.

5. Verfahren nach Anspruch 4,
**dadurch gekennzeichnet,**
**dass** der DW-Stoff während des Umlaufs des DW-Stoff-Trägers (6) ständig durch frischen DW-Stoff dosiert erneuert wird.

6. Verfahren nach Anspruch 1,
**dadurch gekennzeichnet,**
**dass** die Verdunstungstemperatur zwischen 40 und 100° C beträgt.

7. Vorrichtung zur molekularen Verdunstung und Ausbreitung von Duft-/Wirkstoffen über Luftströme in der umgebenden Luft, in geschlossenen Räumen, in Kanälen von Lüftungs- und Klimaanlagen oder dergleichen, mit einer Dosiervorrichtung zum Aufbringen von DW-Stoffen in flüssiger Form auf einen bandförmigen DW-Stoff-Träger (6), der wenigstens teilweise innerhalb einer Ausbreitungszone quer zu seiner Längsachse von einem Luftstrom angeströmt wird und wobei die Dosiervorrichtung außerhalb der Ausbreitungszone angeordnet ist,
**dadurch gekennzeichnet,**
**dass** der bandförmige DW-Stoff-Träger (6) an einem Tragprofil auf Rollen umlaufend bewegbar ist und dass eine Heizvorrichtung vorgesehen ist, welche sich im Wesentlichen längs der Bewegungsbahn des DW-Stoff-Trägers (6) erstreckt.

8. Vorrichtung nach Anspruch 7,
**dadurch gekennzeichnet,**
**dass** der bandförmige DW-Stoff-Träger (6) sowie die zugeordnete Heizvorrichtung wenigstens innerhalb der Ausbreitungszone bis auf Be- und Entlüftungsöffnungen im Inneren eines Hohlprofilträgers (25) gekapselt angeordnet sind.

9. Vorrichtung nach Anspruch 7,
**dadurch gekennzeichnet,**
**dass** der DW-Stoff-Träger (6) als zwischen zwei Umlenkrollen (5, 27) umlaufendes Band ausgebildet ist und
**dass** die Heizvorrichtung in Form eines lang gestreckten Heizstabs (16) zwischen einem oberen (14) und einem unteren Trum (15) des umlaufenden Bands angeordnet ist.

10. Vorrichtung nach Anspruch 9,
**dadurch gekennzeichnet,**
**dass** eine Umlenkrolle (5) außerhalb der Ausbreitungszone als Antriebsrolle ausgebildet und von einem daran angekoppelten Elektromotor (4) angetrieben ist.

11. Vorrichtung nach Anspruch 7,
**dadurch gekennzeichnet,**
**dass** der DW-Stoff-Träger (6) als umlaufendes Band aus eng gebundenen Metallfäden mit hoher Kapillarwirkung besteht.

12. Vorrichtung nach Anspruch 8,
**dadurch gekennzeichnet,**
**dass** der Hohlprofilträger (25) an gegenüberliegenden Seiten schmale Schlitzöffnungen (20, 21) aufweist.

13. Vorrichtung nach Anspruch 12,
**dadurch gekennzeichnet,**
**dass** der Hohlprofilträger (25) aus Teilprofilen (7, 18) zusammengesetzt ist.

14. Vorrichtung nach Anspruch 13,
**dadurch gekennzeichnet,**
**dass** ein oder mehrere Teilprofile (7) schlitzförmige Öffnungen (21) aufweisen.

15. Vorrichtung nach Anspruch 13,
**dadurch gekennzeichnet,**
**dass** die Teilprofile (7, 18) in einem U-förmigen Außenprofil (23) aufgenommen sind, welches ein- oder beidseitig strömungsgünstige Profilansätze (24) aufweist.

16. Vorrichtung nach Anspruch 15,
**dadurch gekennzeichnet,**
**dass** zwischen den Teilprofilen (7, 18) und dem Außenprofil (23) ein U-Profil (26) aus wärmeisolierendem Material eingesetzt ist.

17. Vorrichtung zum Erzeugen eines Türluftschleiers mit einem Blasstrom etwa parallel zu einer Türöffnung, der durch ein Gitter aus einem Strömungskanal für Zuluft austritt, welches sich längs eines Abschnitts des Türrahmens erstreckt, **gekennzeichnet durch** eine ganz oder teilweise außerhalb oder innerhalb des Strömungskanals angeordnete Vorrichtung zur Verdunstung und Ausbreitung von Duft- und/oder Wirkstoffen gemäß einem oder mehreren der Ansprüche 7 bis 16, welche von der Zuluft im Strömungskanal quer zu Ihrer Längsachse angeströmt wird.
